# EUROPEAN PATENT SPECIFICATION

(11) **EP 2 213 231 B1**
(45) Date of publication and mention of the grant of the patent: **04.01.2012**
(21) Application number: 10000907.5
(22) Date of filing: 29.01.2010
(51) Int. Cl.: A61B 5/151

(54) **Integrated body fluid meter and lancing device**
Integrierter Körperflüssigkeitsmesser und Stechhilfevorrichtung
Mesure de fluide de corps intégré et dispositif de lancement

(30) Priority: 30.01.2009 US 362983
(43) Date of publication of application: 04.08.2010
(73) Proprietor: Roche Diagnostics GmbH, 68305 Mannheim (DE); F.Hoffmann-La Roche AG, 4070 Basel (CH)
(72) Inventor: Groll, Henning, Tuscon, AZ 85737 (US); Sauers, Mattew Carlyle, Indianapolis IN 46239 (US); Wightman, Craig, Bristol BS10 6TD (GB); Cottle, David, Backwell BS48 3NL (GB); Walker, Thomas, Redland Bristol BS6 6HL (GB)
(74) Representative: Wildschütz, Sabine

(56) References cited:
- EP-A1- 1 362 551
- EP-A2- 1 779 780
- EP-A2- 1 950 562
- WO-A2-2006/065754
- US-A1- 2007 239 188

## Description

The present invention relates to body fluid meters and lancing devices for obtaining a body fluid sample for testing. In particular, the present invention relates to an integrated blood testing meter and lancing device.

An exemplary body fluid meter includes a blood testing meter such as a blood glucose meter. Blood glucose meters are used by diabetics to monitor their blood glucose levels. Various blood glucose meters are known. A typical blood glucose meter is a hand held device that includes a display, various navigation buttons, a slot or other opening for receiving a test element such as a test strip, and electronics for determining the user's blood glucose level from a sample applied to the element and for otherwise operating the meter.

A typical test element for a body fluid meter includes a test medium and an application point for receiving a fluid sample and transmitting it (for example by capillary action) to the test medium. The test element is sized and shaped to fit within the slot or opening in the meter. A test element comprising a test strip is generally planar and generally rectangular in appearance. Exemplary test strips include the ACCU-CHEK® Aviva test strip and the ACCU-CHEK® Compact Plus test strip, distributed by Roche Diagnostics Corporation of Indianapolis, Indiana. Test elements may also be generally referred to herein as test strips, for purposes of illustration only.

A typical body fluid having parameters and/or characteristics that are of interest to health care providers is blood. Typically, blood samples are tested for such things as glucose or ketone concentrations, as well as certain coagulant properties. A blood sample to be tested is typically obtained by utilizing a lancing device. Various lancing devices are known. In general, the lancing device will include a movable lancet located within a housing, a priming mechanism for placing the lancet in a primed position ready for firing and a firing mechanism for causing the lancet to at least briefly extend from the housing to prick the patient's skin so that a drop of blood can be obtained.

A typical test on a blood sample is conducted by turning on the blood testing meter, inserting a test element into the slot or opening, using the lancet to obtain a blood drop and applying the blood drop to the receiving area of the test element. The electronics in the meter determine the concentration of the analyte of interest, e.g. glucose or ketones or hematocrit, or a particular blood characteristic such as coagulation, and display a test result on the display. Typically, the meter will determine the concentration or characteristic through either an electrochemical analysis or an optical reflectance analysis. Examples of blood testing meters, specifically blood glucose meters and their associated electronics, test elements and lancing devices are disclosed, for example, in U.S. Patent Nos. 7,247,144; 6,969,359; 6,878,120; 6,866,675; 6,793,633; 6,662,439; 6,659,966; 6,645,368; 6,602,268; 6,485,439; 6,419,661; 5,997,817; 5,438,271; 5,366,609; 5,352,351; 5,053,199; 4,999,582; 4,924,879; 4,891,319; Re. 36,268 and Re. 35,803.

In one embodiment of the present invention, a medical testing device includes a blood testing meter having a housing and a lancing device. The lancing device includes a lancet and a tip moveable from a first position in which a portion of the tip extends from the housing to a second position in which a portion of the tip is located at least partially within the housing. In one embodiment, the lancing device is primed upon movement of the tip from the second position to the first position. In another embodiment, other means are provided with the medical testing device for priming the lancing device. The housing includes an opening for receiving a test strip. Inserting a test strip into the opening causes the tip to move from the second position to the first position while remaining in a primed state. The lancet is fired by depressing a button provided for firing the lancet. Alternatively, the tip can be depressed in the direction of the housing when the tip is in the second position. An indicator may be included for indicating the priming status of the lancing device. The device may include a cartridge containing at least two lancets located at least partially within the housing and an opening in the tip through which the lancet extends when it is fired. In one embodiment, the cartridge moves after a lancet is fired to align an unused lancet with the opening. In another embodiment, the tip moves after a lancet is fired to align an unused lancet with the opening. In yet another embodiment, alignment with respect to the tip and an unused lancet is conducted manually. An indicator can be provided to display the number of used or unused lancets in the cartridge.

In other embodiments of the invention, a medical testing device includes a blood glucose meter having a housing, a lancing device located at least partially within the housing and including a lancet movable from a first position located at least partially within the housing to a second position located at least partially outside the housing and means for priming the lancing device for movement from the first position to the second position and back to the first position. The means for priming the lancing device can take various forms, such as a tip on the lancing device movable from a first position to a second position, a movable cover, a lever, a movable display or a button. In other embodiments, inserting a test strip into the meter primes the lancing device.

The above-mentioned and other features of this invention and the manner of obtaining them will become more apparent and the invention itself will be better understood by reference to the following description of embodiments of the present invention taken in conjunction with the accompanying drawings, wherein:
Figures 1-4 illustrate an integrated blood glucose meter and lancing device according to one embodiment of the present invention.
Figures 5-7 illustrate another embodiment of an integrated blood glucose meter and lancing device according to the present invention.
Figures 8-10 illustrate another embodiment of an integrated blood glucose meter and lancing device according to the present invention.
Figures 11-12 illustrate another embodiment of an integrated blood glucose meter and lancing device according to the present invention.
Figure 13 illustrates another embodiment of an integrated blood glucose meter and lancing device according to the present invention.
Figure 14 illustrates another embodiment of an integrated blood glucose meter and lancing device according to the present invention.
Figures 15-16 illustrate another embodiment of an integrated blood glucose meter and lancing device according to the present invention.
Figure 17 illustrates another embodiment of an integrated blood glucose meter and lancing device according to the present invention.
Figures 18-19 illustrate another embodiment of an integrated blood glucose meter and lancing device according to the present invention.
Figures 20A-20B illustrate another embodiment of an integrated blood glucose meter and lancing device according to the present invention.
Figure 21A-21B illustrate another embodiment of an integrated blood glucose meter and lancing device according to the present invention.
Figure 22 illustrates another embodiment of an integrated blood glucose meter and lancing device according to the present invention.
Figure 23 illustrates another embodiment of an integrated blood glucose meter and lancing device according to the present invention.
Figure 24 illustrates another embodiment of an integrated blood glucose meter and lancing device according to the present invention.
Figure 25 illustrates another embodiment of an integrated blood glucose meter and lancing device according to the present invention.
Figure 26 illustrates another embodiment of an integrated blood glucose meter and lancing device according to the present invention.
Figure 27 illustrates another embodiment of an integrated blood glucose meter and lancing device according to the present invention.
Figure 28 illustrates another embodiment of an integrated blood glucose meter and lancing device according to the present invention.
Figure 29 illustrates another embodiment of an integrated blood glucose meter and lancing device according to the present invention.
Figure 30 illustrates another embodiment of an integrated blood glucose meter and lancing device according to the present invention.
Figure 31 illustrates another embodiment of an integrated blood glucose meter and lancing device according to the present invention.
Figures 32-33 illustrate another embodiment of an integrated blood glucose meter and lancing device according to the present invention.
Figures 34-35 illustrate another embodiment of an integrated blood glucose meter and lancing device according to the present invention.
Figure 36-37 illustrate another embodiment of an integrated blood glucose meter and lancing device according to the present invention.

Corresponding reference characters indicate corresponding parts throughout the several views. Although the drawings represent embodiments of various features and components according to the present invention, the drawings are not necessarily to scale and certain features may be exaggerated in order to better illustrate and explain the present invention. The exemplifications set out herein illustrate embodiments of the invention, and such exemplifications are not to be construed as limiting the scope of the invention in any manner.

For the purposes of promoting an understanding of the principles of the invention, reference will now be made to the embodiments illustrated in the drawings, which are described below. No limitation of the scope of the invention is thereby intended. The invention includes any alterations and further modifications in the illustrated devices and described methods and further applications of the principles of the invention which would normally occur to one skilled in the art to which the invention relates.

Each of the following embodiments of the invention includes a blood testing meter integrated with a lancing device. The internal electronics of the blood testing meter can take any form and/or perform any function sufficient to properly determine concentration of an analyte of interest, e.g. glucose or ketones or hematocrit, or a particular blood characteristic such as coagulation, and, in most embodiments, display a test result on the meter's display. For purposes of simplicity and efficiency, the embodiments of the invention will be described typically as comprising blood glucose meters for determining concentration of glucose. It will be understood, however, that such description is directly analogous to blood testing meters configured for other types of blood testing as have been described herein by way of example, and such other blood testing meters are considered to be within the scope of the present invention. Furthermore, for purposes of the invention, it does not matter whether the blood testing result is determined by an electrochemical process, an optical reflectance process or another process. The blood testing meter can include features in addition to testing of blood and the display of results. For example, the blood testing meter can include a memory for storing past blood testing results readings, a journal or diary for entering comments relevant to the user's medical treatment or activities, or other features. The meter can also include electronics for communicating with other devices, such as personal computers, either through a physical connection such as a USB port or through a wireless connection. Similarly, the type of test strip utilized is not a feature of the invention.

In embodiments described below, the lancing device can be moved relative to the remainder of the testing meter. The lancing device also must be primed so that the lancet can be fired (i.e., extended from the lancing device to prick the user's skin). The lancing device integrated with the blood testing meter can utilize any one of a number of internal mechanisms for movement and/or priming. For example, various linear and/or rotary mechanisms, such as springs (linear, leaf, rotational, coil or other), manual slides or actuators, motor drives, a worm and rack, a rack and pinion, helical drives, solenoids, electromagnetic drives or other mechanisms can be used. Note also that various lancet devices can be used with the lancing mechanism. For example, single load lancets as shown in U.S. Patent Nos. 6,969,359 and 6,602,268 can be used with embodiments of the present invention. A cartridge, such as a drum containing multiple lancets, can also be utilized. Examples of such lancing devices are the ACCU-CHEK® Multiclix® and ACCU-CHEK® Softclix® lancing devices sold by Roche Diagnostics Corporation and disclosed in U.S. Patent Nos. 4,924,879; 5,318,584; RE35,803; 7,077,828; 6,419,661; 7,223,276; and U.S. Pat. Pub. Nos. 2003/0153939; 2004/0034318; and 2004/0260325.

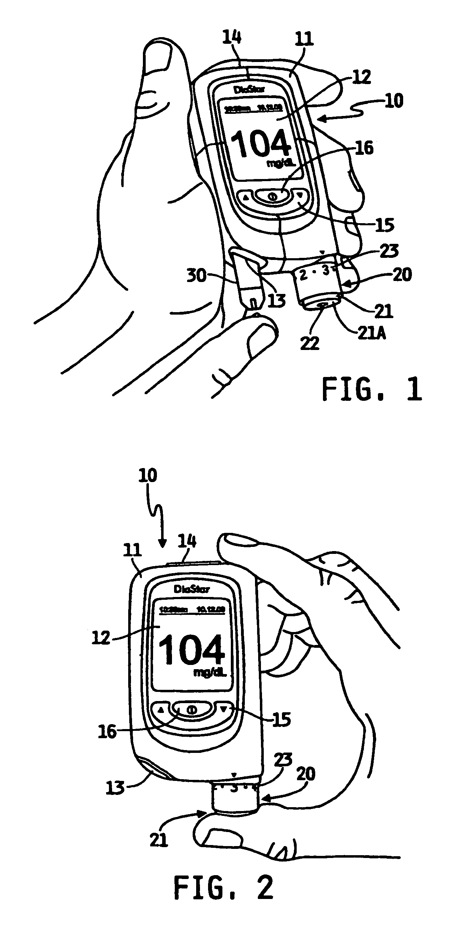

Figure 1 shows a perspective view of a medical testing device comprising an integrated blood glucose meter and lancing device according to one embodiment of the present invention. Meter 10 generally includes a housing 11, a display 12, a test strip opening 13, a firing button 14, navigation buttons 15 and a power button 16. Navigation buttons 15 are used to access various features of the meter 10, such as the memory function, journal, set up functions and other features.

Lancing device 20 is at least partially located in housing 11 and partially extends therefrom. Lancing device 20 includes a tip 21 having a first end 21A for contacting the user's skin, an opening 22 through which a lancet (not shown) extends upon firing and a depth gauge 23. One or more lancets are located in lancing device 20. In one embodiment, a cartridge (such as a substantially cylindrical drum) containing multiple lancets is located in lancing device 20. In other embodiments, a single lancet is located in lancing device 20. If multiple lancets are located in lancing device 20, the invention includes means for aligning an unused lancet with opening 22 after a lancet has been fired, as described below. Use of depth gauge 23 is well known in the art and is disclosed, for example, in U.S. Patent No. 6,419,661.

Depth gauge 23 is used to set the penetration depth of the lancet into the user's skin. The integrated blood glucose meter and lancing device in Figure 1 is shown with a test strip 30 inserted in opening 13.

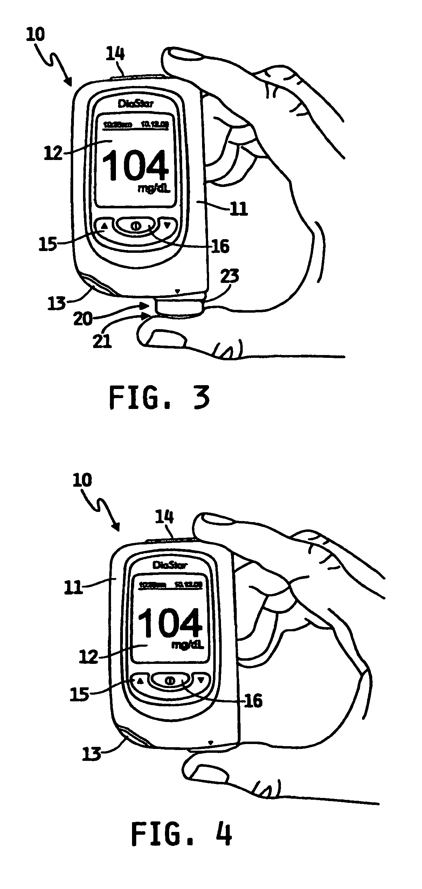

Figure 2 shows the integrated blood glucose meter and lancing device with the depth gauge 23 set to the desired penetration depth prior to priming the lancet for firing. Figure 3 shows the lancing device 20 in the primed position. In this position, the user has pushed a portion of tip 21 into housing 11 of meter 10, which cocks or primes the internal firing mechanism (not shown) and lancet for use. Various priming mechanisms can be used, such as those disclosed in the lancing device patents referred to above. To conduct a test, the user positions end 21A of lancing device 20 against his or her skin at the desired testing location and depresses button 14 to fire or eject the lancet from opening 22 and into the skin. After firing, the lancet moves back into tip 21 so as to prevent the user or others from accidentally contacting the used lancet. In embodiments of the invention that include a cartridge or drum with multiple lancets, the lancing device 20 is configured to align an unused lancet with opening 22 after firing. In one embodiment, the cartridge or drum moves, such as by translation or rotation, to align an unused lancet with opening 22. In other embodiments, tip 21 moves, as by rotation or translation, to align an unused lancet with opening 22. Movement of the cartridge or tip 21 can be accomplished automatically or manually (as described below). Furthermore, movement of the cartridge or tip 21 can be achieved mechanically, electrically or by a combination of mechanical and electrical devices.

After the lancet is fired, a drop of blood is applied to test strip 30 which is inserted into opening 13 as shown in Figure 1. The blood glucose meter 10 will then determine the blood glucose reading and display it as is known in the art. After the test is complete, the lancing device 20 can be placed in a storage position by depressing tip 21 substantially completely into housing 11 as shown in Figure 4. The storage position can also maintain the lancing device 20 in the primed position ready for firing.

In an alternative embodiment, a test strip 30 is inserted into opening 13 and causes tip 21 to extend from housing 11. In this embodiment, lancing device 20 is primed and fired only after the test strip 30 is inserted. Note that test strip opening 13 and lancing device 20 are positioned such that the lancing device 20 can be used without interference from the test strip 30. Similarly, the user can apply blood to test strip 30 without interference from lancing device 20. This can be achieved, for example, by configuring the meter such that the end of tip 21 and the end of test strip 30 lie in essentially the same plane.

In this embodiment (and those described below) the testing meter may also include a safety mechanism to prevent accidental firing of the lancet until a certain condition is met, such as insertion of a test strip. This allows transport and storage of the device in a primed state. The safety mechanism can include a catch and a release trigger. The catch can have a rotational or translational trigger. These triggers release the lancing mechanism from it storage state to allow lancing. The catch can be either a linear or rotational catch such as a simple lever, block or rotary catch which prevents the lancing mechanism from firing until it is moved into the lancing position. Examples of linear release triggers include, but are not limited to a lever or cantilever, a manual slide, a motor drive, a worm and rack, a pinion and rack and/or a magneto-motive drive, such as a solenoid or electromagnet. Examples of rotary release triggers include, but are not limited to, a wheel or cam, a motor drive where the output motion of the release is rotational, a helical mechanical drive, where the input motion is rotary (from, for example, a crank, wheel, lever or a pinion or worm) and/or a magneto-motive drive with rotational output.

The testing meter can also include various feedback sensors that provide information about the state of the mechanical elements of the system, such as the position of the lancing device (extended or retracted), the state of lancing mechanism (primed or not primed), the number of lancets used and/or remaining and the number of times that a given lancet has been used. Examples of sensors that can be used include, but are not limited to, encoders, photo-sensors, switches, photo-detectors, photo-interrupters and magnetic or electromagnetic devices, such as Hall effect sensors. The sensors can provide output of the information on an electronic display or can cause a change in a mechanical indicator, such as a number on a rotating component.

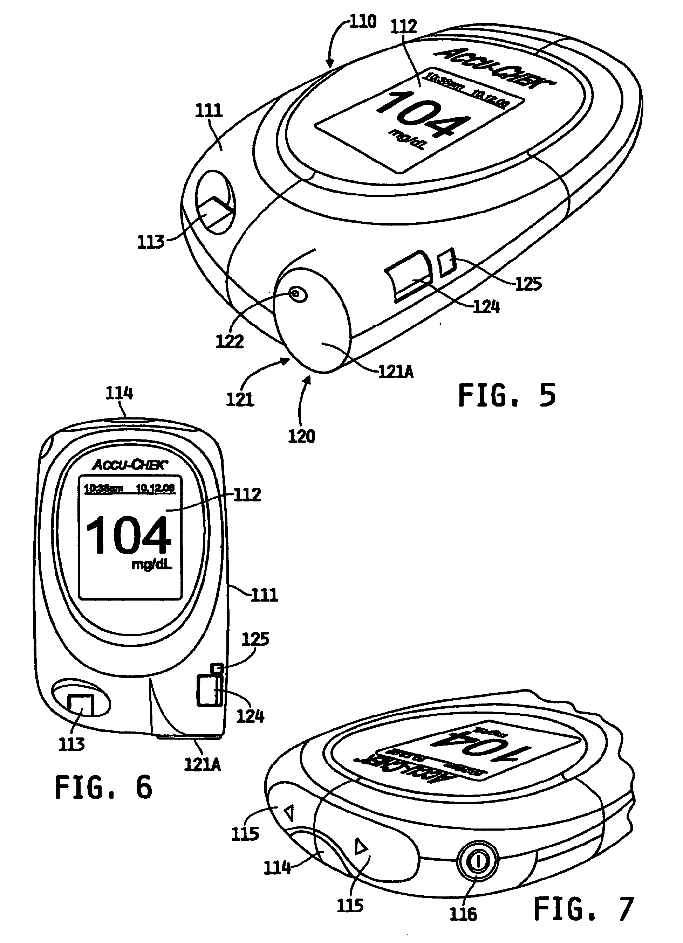

Figures 5-7 show another embodiment of an integrated blood glucose meter and lancing device according to the present invention. In this embodiment, navigation buttons 115 and power button 116 are located near the end of the meter 110 opposite opening 113. This embodiment further includes an actuator 124 that can be used to manually advance the lancet cartridge or drum located within lancing device 120. Alternatively, the rotary drum can advance automatically as described in conjunction with the embodiment of Figures 1-4.

The embodiment of Figures 5-7 also includes a status indicator 125 that provides information about the status of lancing device 120. For example, status indicator 125 can change from one color to another if lancing device 120 is in the primed position. This can be done either electronically or manually. Status indicator 125 can take several forms. For example, status indicator 125 can be a dear window with a colored member visible therethrough. The member can move as the status of lancing device 120 changes, thereby changing the color visible through the window. Alternatively, status indicator 125 could be a light that either changes color or turns on and off in response to the status of lancing device 120. In other embodiments, status indicator 125 can display a number indicating either the remaining unused lancets in lancing device 120 or the number of the lancets that have been used. The lancing device of the embodiment shown in Figures 5-7 includes an extendable tip 121 like the one shown and described in conjunction with the embodiment of Figures 1-4. As with the tip 23 illustrated in the first embodiment, tip 121 of this embodiment can be extended from the storage position by pressing inward on end 121A. In an alternative embodiment, tip 121 extends automatically upon insertion of a test strip into opening 113.

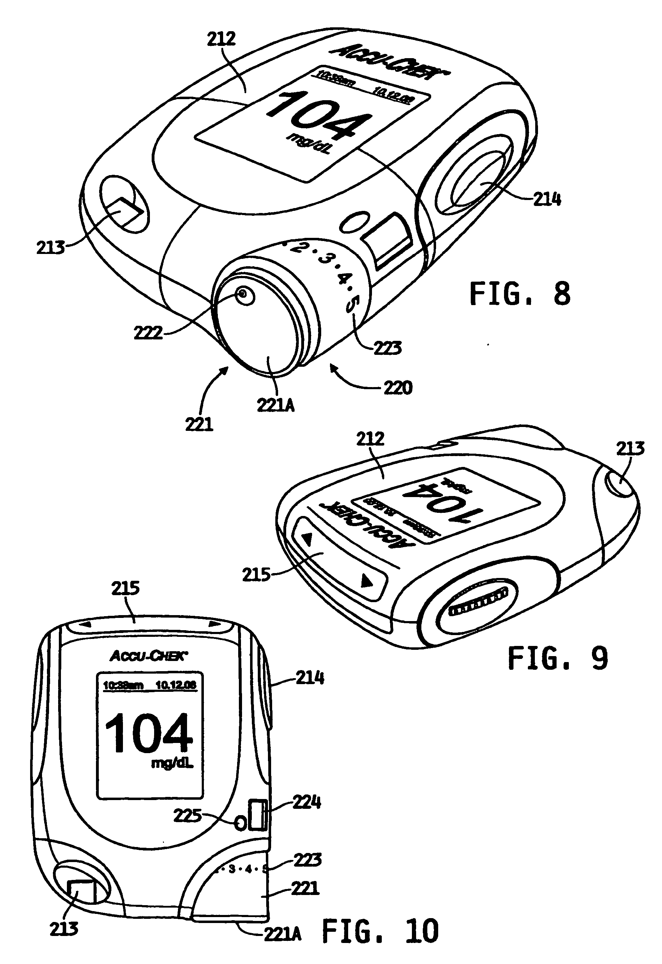

Figures 8-10 illustrate another embodiment of the invention. In this embodiment, tip 221 of lancing device 220 cannot move in and out of housing 211. Rather, it either remains stationary in one embodiment of the invention or, alternatively, rotates to align opening 222 with an unused lancet, as described in connection with the embodiments discussed above. One of several priming and firing mechanisms can be utilized with this embodiment. For example, firing button 214 can be a triple action button in which depressing button 214 once causes it to pop out or extend from a stored position on the side of blood glucose meter 10. Depressing it a second time primes the firing mechanism of the lancing device 220 and depressing it a third time fires the lancet. Alternatively, button 214 can be a double action button that remains in the extended position during storage. In that embodiment of the invention, depressing button 214 once fires the lancet. Depressing button 214 a second time primes the lancing device for the next use.

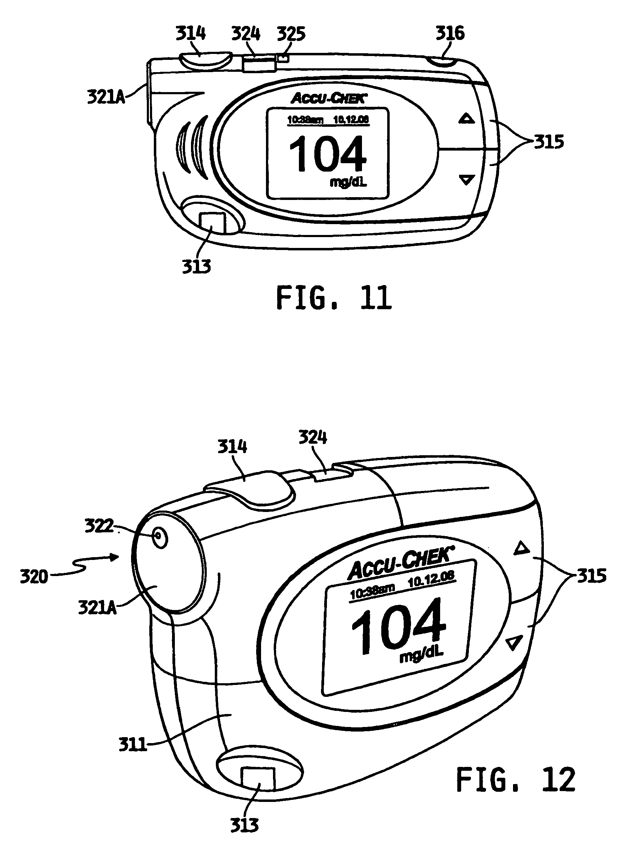

Figures 11 and 12 show another embodiment of the present invention. This embodiment is similar to that shown in Figures 5-7 except that the locations of navigation buttons 315 and firing button 314 have been moved. Furthermore, the locations of lancing device 320 and strip opening 313 have been switched.

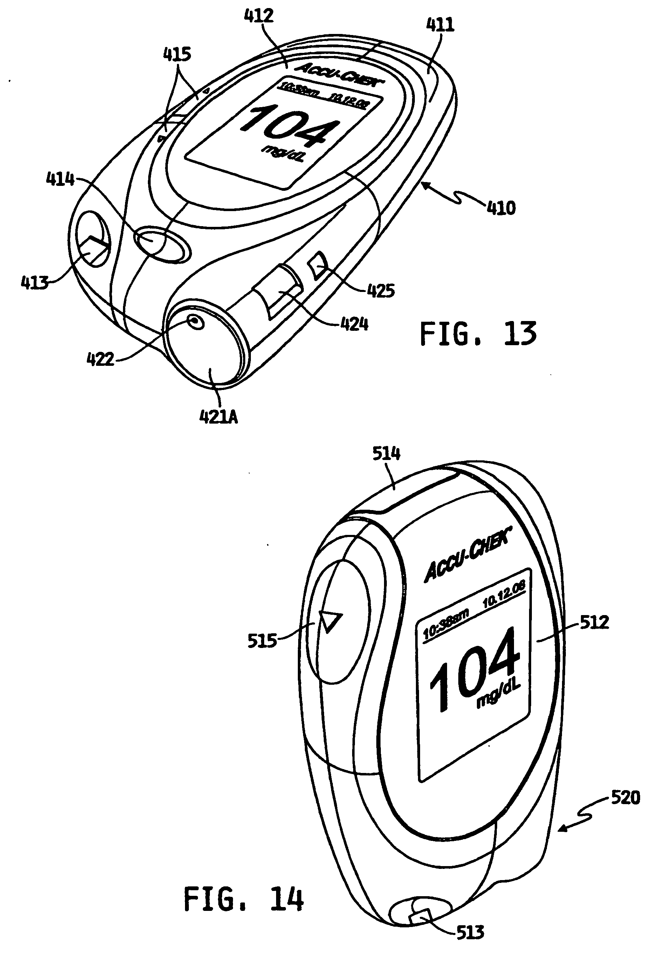

Figure 13 shows an embodiment of the invention in which the firing button 414 is located on the front of the meter 410 below display 412. Navigation buttons 415 are located on one side of display 412.

Figure 14 shows another configuration for an integrated blood glucose meter and lancing device according to the present invention. In this embodiment, firing button 514 is located at one end of meter 510 above display 512. A single navigation button 515 is located on the side of meter 510 near the same end.

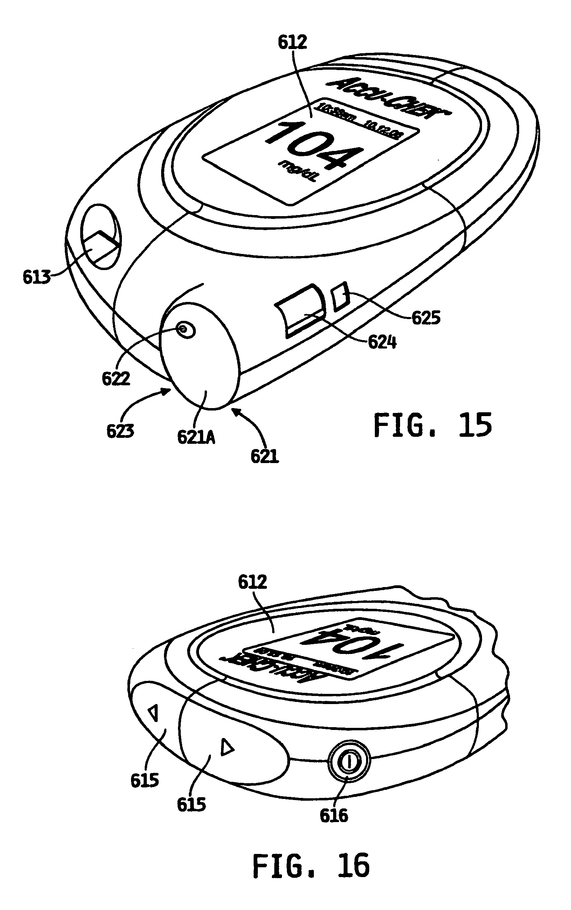

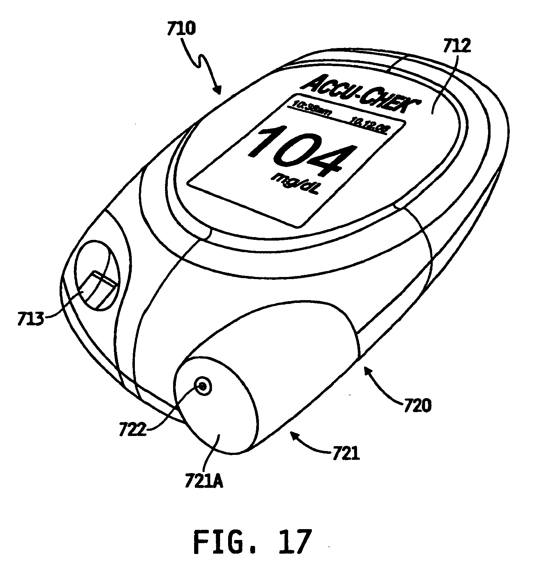

Figures 15, 16 and 17 show embodiments of the invention that do not include a firing button. Instead, the lancet is fired by pressing against the extendable tip 621 (Figures 15 and 16) or 721 (Figure 17).

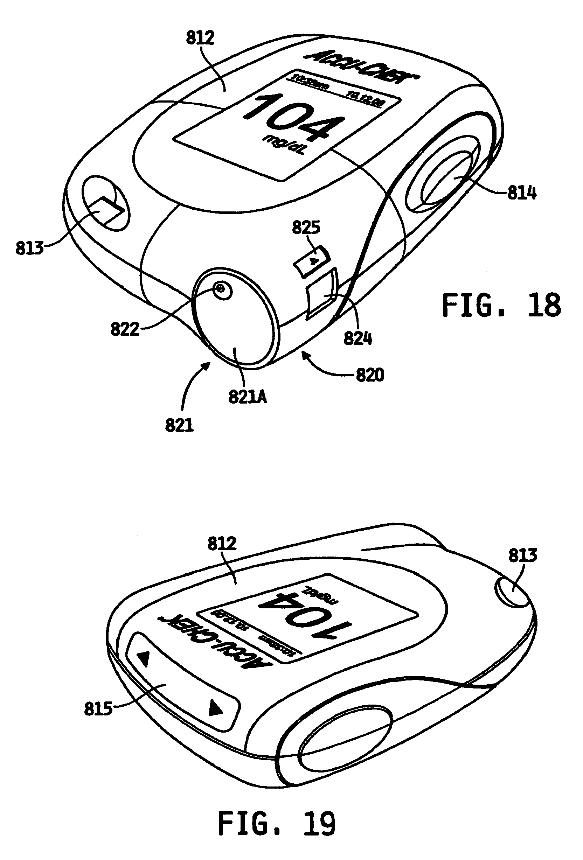

Figures 18 and 19 show an additional embodiment with an extendable firing button 814 similar to the one shown in Figures 8-10. However, in this embodiment, in addition to performing the priming and firing functions described above, depressing button 814 causes tip 821 to extend from lancing device 820.

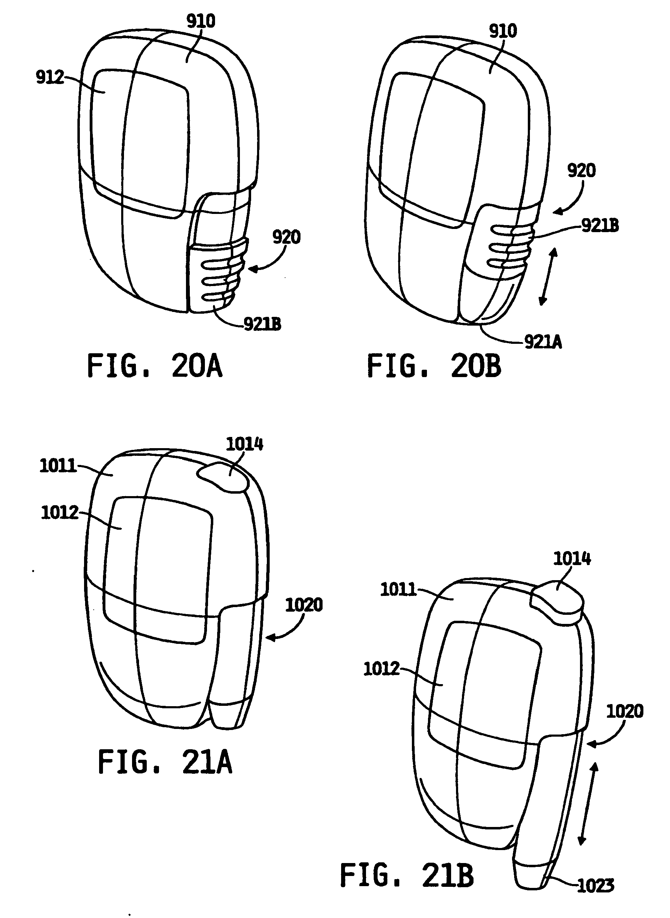

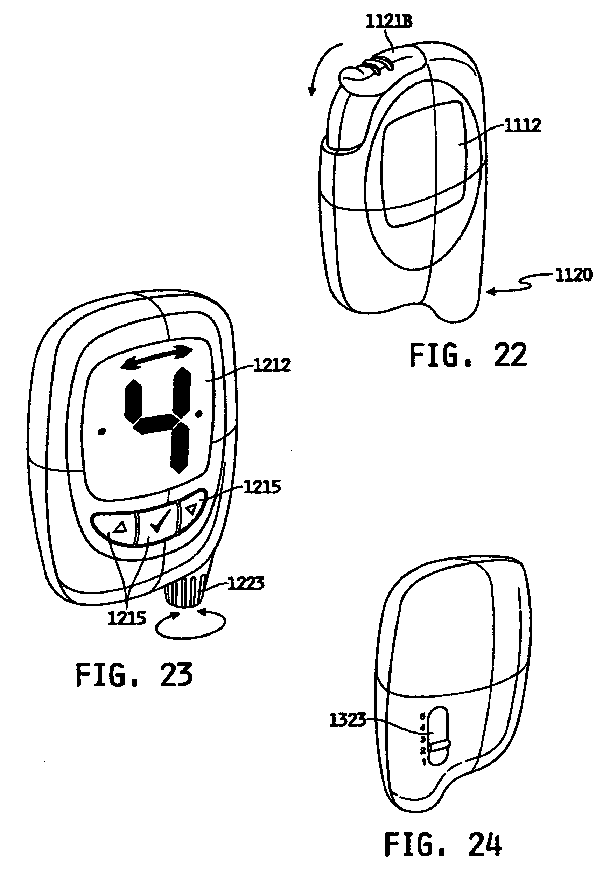

Figures 20A, 20B, 21A, 21B and 22 show additional mechanisms for priming a lancing device integrated with a blood glucose meter. In Figures 20A and 20B, a sliding cover 921B is moved from an initial position (Figure 20A) to a second position (Figure 20B) to prime the lancing device 920. In Figures 21A and 21B, the lancing device 1020 is primed by either pulling or pushing it in an axial direction. Note that as shown optionally, priming the lancing device 1020 in the embodiments of Figures 21A and 21B causes firing button 1014 to extend from housing 1011. In the embodiment of Figure 22, lancing device 1120 is primed by sliding actuator 1121B from a first position to a second position in a manner similar to that of the embodiment of Figures 20A and 20B.

Figure 23 shows an embodiment of the invention in which the priming mechanism, firing mechanism, lancet advancement mechanism and depth setting mechanism can all be electronically controlled. This can be accomplished with a motor, a solenoid a piezo electric device or other mechanism. In the embodiment shown, the depth setting is displayed on the display 1212. As noted above, other information, such as the number of lancets used, the number of lancets remaining, the number of times a lancet has been used and/or the state of the lancing device (either primed or unprimed) can also be indicated on display 1212.

Figure 24 shows an embodiment of the invention in which a sliding actuator 1323 is used to set the depth of the lancet penetration.

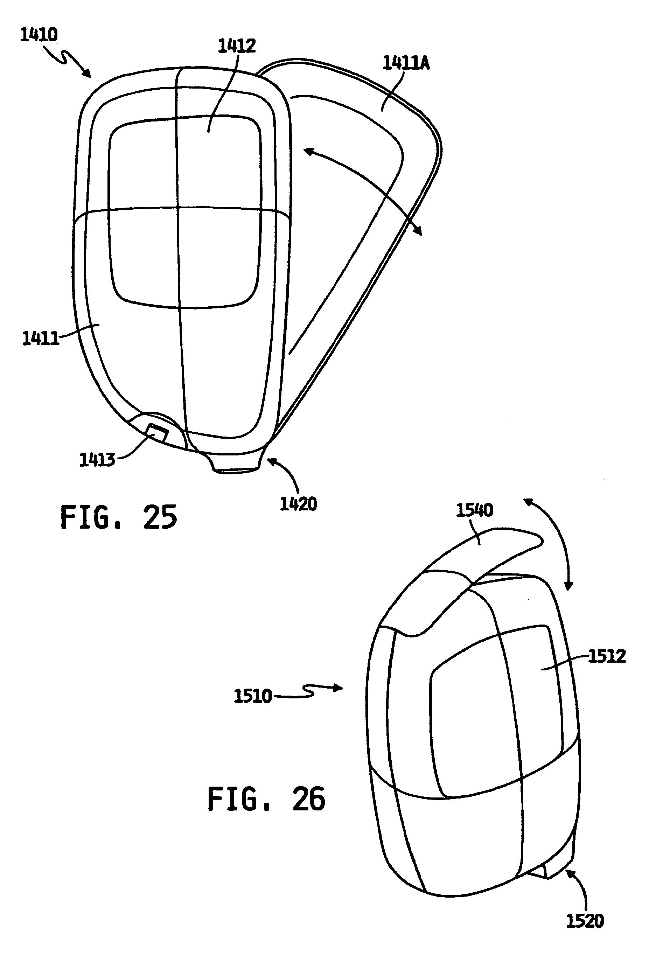

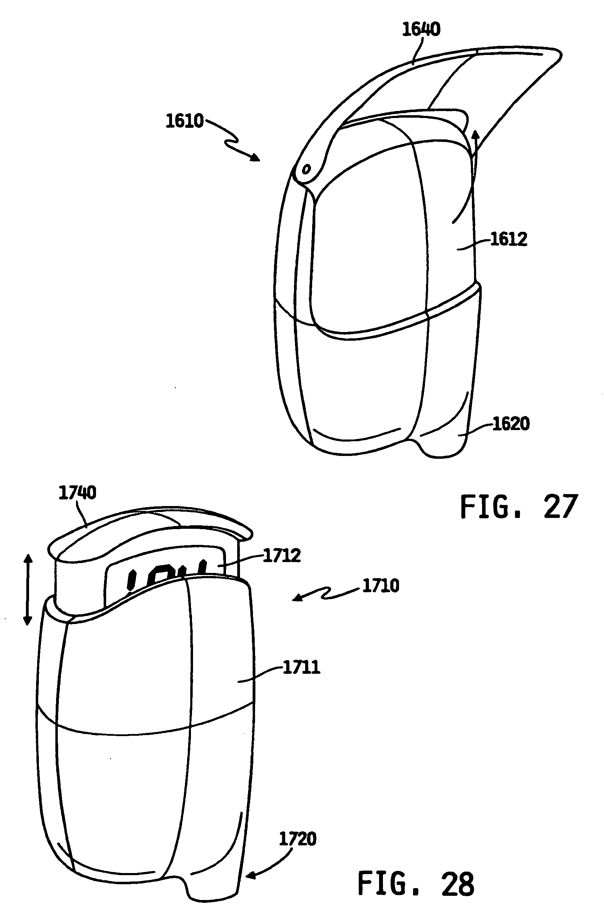

Figures 25-27 show additional mechanisms for priming the lancet. In the embodiment of Figure 25, meter 1410 includes a hinged back cover or activation lever 1411A, which is shown as being coupled to the meter housing. Lancing device 1420 is primed by rotating the lever 1411A relative to the meter, utilizing, e.g., an internal ratchet and helical drive mechanism (not shown) actuated by the rotation of the level about a pivot or fulcrum connection to the meter housing. The lever 1411A may rotate outwardly to the side, away from the housing, and be returned into place, thus priming the lancing device. Alternatively, the rotation of the cover may reveal, e.g., a test strip storage compartment or other useful aspect of the system that may be concealed when the cover is in place. Priming actuation is typically mechanically translated from the rotational displacement about the pivot connection such as with a rack-and-pinion with the angular rack placed on the lever and the pinion priming the lancet. Figure 26 shows a blood glucose meter 1510 with a rotary cam, linkage drive, or linear priming lever 1540 for priming lancing device 1520. Figure 27 shows a hinged flap or cover 1640 that is similarly used to prime lancing device 1620. Note that cover 1640 can be used to cover and protect display 1612 when meter 1610 is not in use. In an alternative embodiment, cover 1640 could slide over display 1612 to prime lancing device 1620. Display 1612 could also be located on the opposite side of blood glucose meter 1610.

Figure 28 shows an embodiment in which display 1712 is stored within housing 1711 when meter 1710 is not in use. Lancing device 1720 is primed by grasping the top end 1740 of display 1712 and extending it from housing 1711. Alternatively, display 1712 can be spring loaded in a manner similar to the extendable lancing device tips described in connection with embodiments discussed above. In this embodiment, pushing in on end 1740 will cause display 1712 to move slightly farther into housing 1711 before releasing and extending from housing 1711. This also provides the mechanical energy for movement of the lancing mechanism. In yet another embodiment, inserting a test strip into meter 1710 can cause display 1712 to extend from housing 1711.

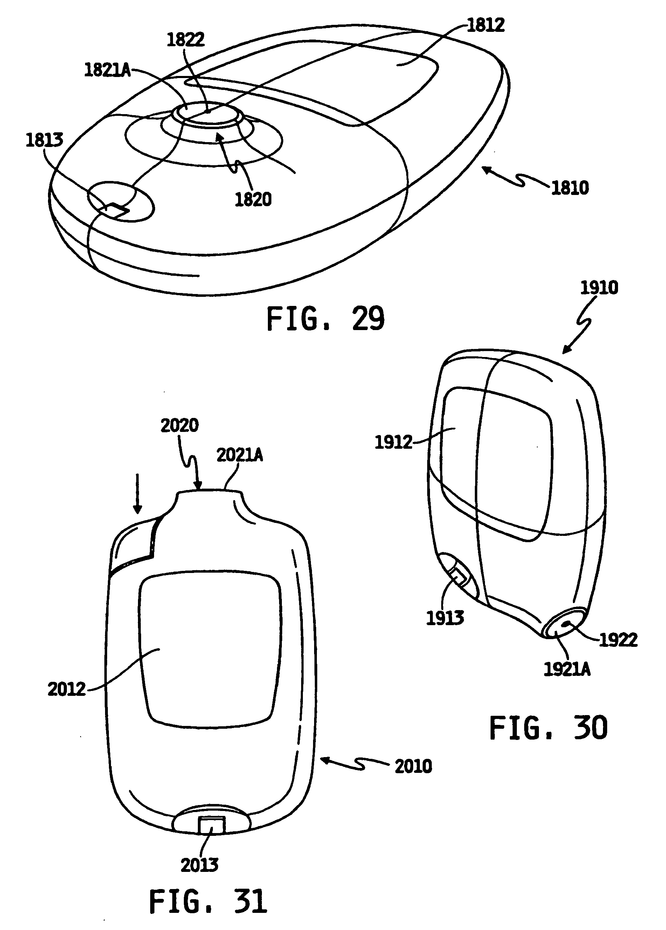

Figures 29-31 show embodiments in which the lancing device is located at different positions on the blood glucose meter. In Figure 29, the lancing device 1820 is positioned such that tip surface 1821A faces the same direction as display 1812. In Figure 30, tip surface 1921A is directed outwardly from a corner of meter 1910. Lancing mechanism 2020 is located above display 2012 opposite test strip opening 2013 in the embodiment of Figure 31. In each of these embodiments (as with the other embodiments of the invention) the locations of the test strip opening and lancing device are such that the user can conduct the lancing operation without interference from an inserted test strip and apply blood to the test strip without interference from the lancing device.

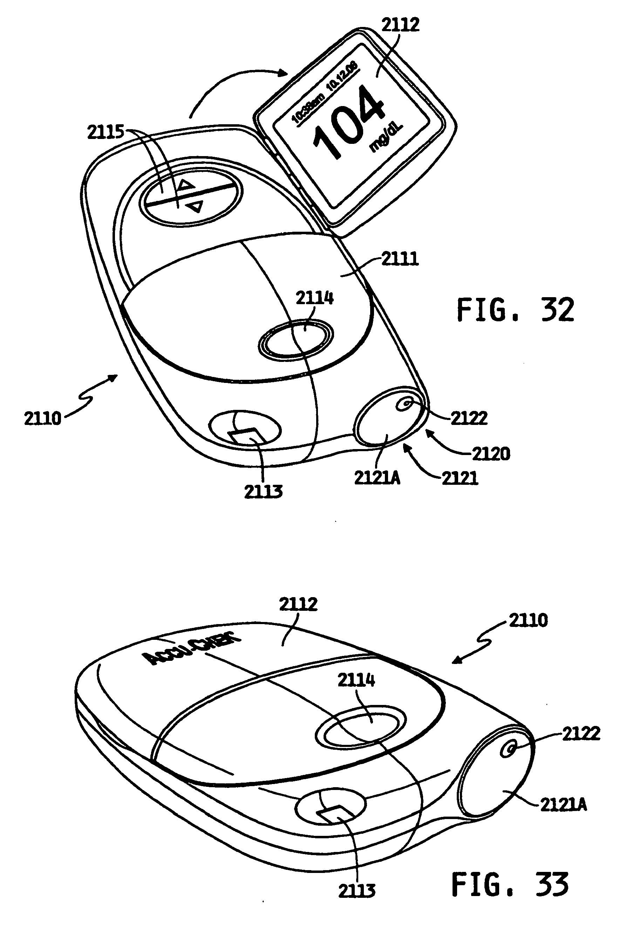

Figures 32 and 33 show another embodiment of an integrated blood glucose meter and lancing device according to the present invention. In this embodiment, display 2112 is attached to housing 2111 by a hinge and can pivot outwardly therefrom. In the closed position (Figure 33), display 2112 covers navigation buttons 2115. As with other embodiments of the invention, various priming, lancet advancement and firing mechanisms can be utilized. In one embodiment, lancing device 2120 includes an extendable tip 2121 that extends when a test strip is inserted in opening 2113. The lancet drum may rotate within lancing device 2120 to advance the next unused lancet to the firing position. Alternatively, tip 2121 may rotate. In the embodiment shown in Figures 32 and 33, lancing device 2120 can be primed by opening display 2112 to the position shown in Figure 32.

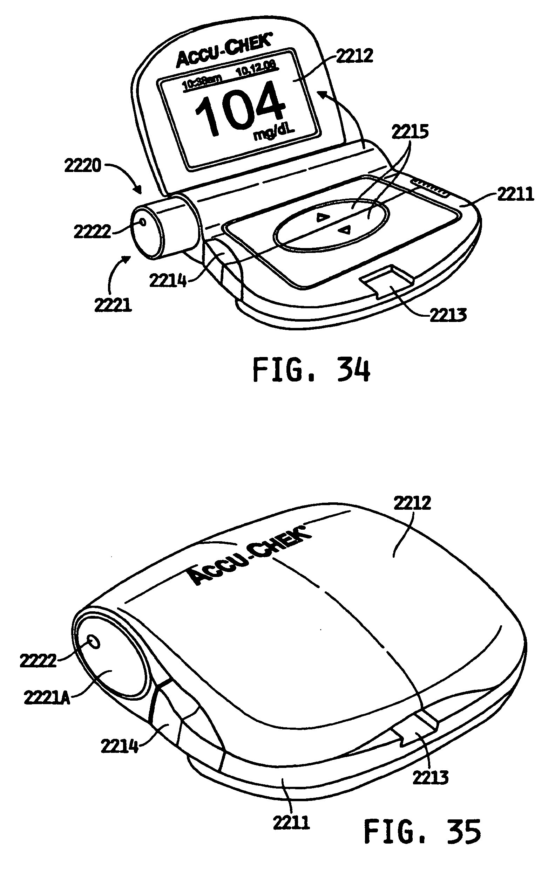

Figures 34 and 35 show another embodiment of an integrated blood glucose meter and lancing device according to the present invention. In this embodiment, display 2212 is attached to housing 2211 by a hinge or similar device and is movable from a position covering navigation buttons 2215 as shown in Figure 35 to an open position shown in Figure 34. The lancing device is primed by moving display 2212 to the open position. For example, moving display 2212 to the open position can activate a cam mechanism that causes translational movement of tip 2221. In an alternative embodiment, dosing display 2212 primes lancing device 2220. Inserting a test strip into test strip opening 2213 causes extendable tip 2221 of lancing device 2220 to extend from the side of the meter 2210. As an alternative, opening display 2212 could cause tip 2221 to extend.

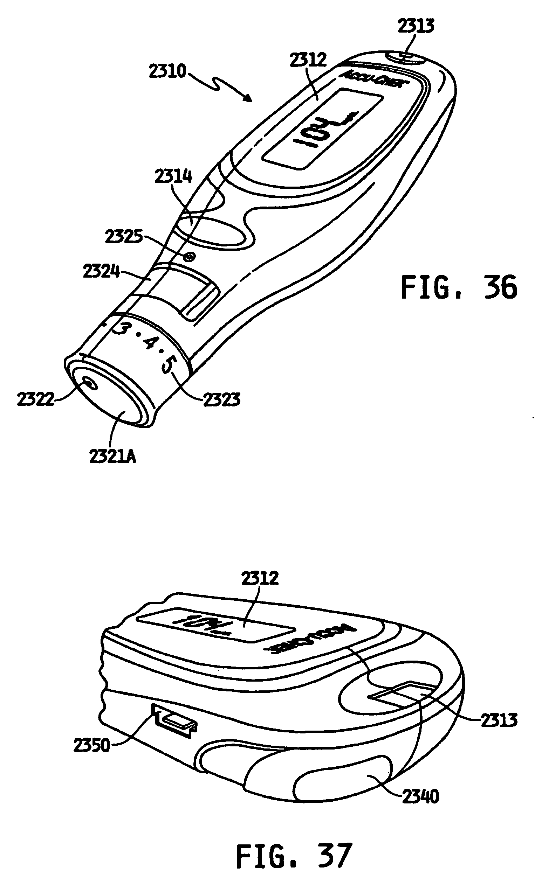

Figures 36 and 37 show another embodiment of an integrated blood glucose meter and lancing device. In this embodiment, the device has a generally elongated, pen-like shape. Meter 2310 is turned on by depressing button 2314. Once powered, button 2314 is used to navigate through information displayed on display 2312. The firing mechanism is primed by depressing button 2340 a first time and fired by depressing button 2340 a second time. Note that this embodiment includes an optional USB port 2350. With this embodiment, data stored in the meter 2310 can be downloaded to a personal computer or other compatible device using port 2350 or any other suitable wired or wireless mechanism for data transfer. Note also that the embodiment of Figures 36 and 37 can be made more compact by eliminating display 2312. In such an embodiment, blood glucose readings would simply be stored in the memory of meter 2310 and would be accessed by connecting meter 2310 to a personal computer or other compatible device through USB port 2350 or other suitable access means.

While the invention has been taught with specific reference to the embodiments described above, one skilled in the art will recognize that changes can be made in form and detail without departing from the scope of the invention. As described above, many of the features are interchangeable. For example, in embodiments in which the lancing device includes an extendable tip, the tip may be extended by depressing in into the device, by inserting a test strip, by pushing a button or other means. Those skilled in the art will recognize that in embodiments in which only one of these methods is disclosed other disclosed methods could be substituted. The described embodiments are to be considered, therefore, in all respects only as illustrative and not restrictive. As such, the following claims, rather than the above description, define and illustrate the scope of the invention.

## Claims

1. A medical testing device, including:
a blood testing meter having a housing;
a lancing device including a lancet and a tip movable from a first position located at least partially within the housing to a second position located at least partially outside the housing; and
means for priming the lancing device for movement of the lancet from the first position to the second position.

2. The medical testing device according to claim 1, wherein the means for priming includes a tip on the lancing device movable from a first position to a second position.

3. The medical testing device according to claim 1, wherein the means for priming includes an opening in the housing for receiving a test element and wherein inserting a test element into the opening primes the lancet.

4. The medical testing device according to claim 1, wherein the means for priming includes a cover movable from a first position on the housing to a second position on the housing.

5. The medical testing device according to claim 1, wherein the means for priming includes a lever movable from a first position to a second position.

6. The medical testing device according to claim 1, further including an indicator for indicating whether the lancet is in the primed or unprimed state.

7. The medical testing device according to claim 6, further including a display and wherein the indicator appears on the display.

8. The medical testing device according to claim 1, wherein the means for priming includes a display movable from a first position located at least partially within the housing to a second position located at least partially outside the housing.

9. The medical testing device according to claim 1, further including a display and wherein the means for priming includes a cover movable from a first position over the display to a second position exposing the display.

10. The medical testing device according to claim 1, wherein the means for priming includes a display movable from a first position facing the housing to a second position.

11. The medical testing device according to claim 10, wherein the means for priming includes a button located on the housing and depressing the button moves it from a storage position to a use position.

12. The medical testing device according to claim 11, wherein depressing the button a second time primes the lancet.

13. The medical testing device according to claim 12, wherein depressing the button a third time fires the lancet.

14. The medical testing device according to claim 1, further including a cartridge containing at least two lancets located at least partially within the housing and an opening in the housing through which the lancet extends when it is in the second position and wherein the cartridge is configured to move after a lancet is fired to align an unused lancet with the opening.

15. The medical testing device according to claim 14, further including an indicator displaying the number of unused and/or used lancets in the cartridge.

## Patentansprüche

1. Medizinische Testvorrichtung mit
einem Bluttestmessgerät mit einem Gehäuse,
einer Lanzettenvorrichtung mit einer Lanzette und einer Spitze, die aus einer ersten Position, die sich mindestens teilweise im Gehäuse befindet, in eine zweite Position, die sich mindestens teilweise außerhalb des Gehäuses befindet, bewegt werden kann, und
einem Mittel zum Spannen der Lanzettenvorrichtung, um die Lanzette aus der ersten Position in die zweite Position zu bewegen.

2. Medizinische Testvorrichtung nach Anspruch 1, wobei das Mittel zum Spannen eine Spitze an der Lanzettenvorrichtung aufweist, die aus einer ersten Position in eine zweite Position bewegt werden kann.

3. Medizinische Testvorrichtung nach Anspruch 1, wobei das Mittel zum Spannen eine Öffnung im Gehäuse zur Aufnahme eines Testelements aufweist und wobei das Spannen der Lanzette durch Einführen eines Testelements in die Öffnung erfolgt.

4. Medizinische Testvorrichtung nach Anspruch 1, wobei das Mittel zum Spannen eine Abdeckung aufweist, die aus einer ersten Position am Gehäuse in eine zweite Position am Gehäuse bewegt werden kann.

5. Medizinische Testvorrichtung nach Anspruch 1, wobei das Mittel zum Spannen einen Hebel aufweist, der aus einer ersten Position in eine zweite Position bewegt werden kann.

6. Medizinische Testvorrichtung nach Anspruch 1, ferner mit einem Anzeiger, um anzuzeigen, ob die Lanzette im gespannten oder ungespannten Zustand ist.

7. Medizinische Testvorrichtung nach Anspruch 6, ferner mit einer Anzeige und wobei der Anzeiger auf der Anzeige erscheint.

8. Medizinische Testvorrichtung nach Anspruch 1, wobei das Mittel zum Spannen eine Anzeige aufweist, die aus einer ersten Position, die sich mindestens teilweise im Gehäuse befindet, in eine zweite Position, die sich mindestens teilweise außerhalb des Gehäuses befindet, bewegt werden kann.

9. Medizinische Testvorrichtung nach Anspruch 1, ferner mit einer Anzeige, und wobei das Mittel zum Spannen eine Abdeckung aufweist, die aus einer ersten Position über der Anzeige, in eine zweite Position, die die Anzeige freilegt, bewegt werden kann.

10. Medizinische Testvorrichtung nach Anspruch 1, wobei das Mittel zum Spannen eine Anzeige aufweist, die aus einer ersten Position, in der sie dem Gehäuse zugewandt ist, in eine zweite Position bewegt werden kann.

11. Medizinische Testvorrichtung nach Anspruch 10, wobei das Mittel zum Spannen einen Knopf aufweist, der am Gehäuse angeordnet ist, wobei der Knopf durch Niederdrücken aus einer Lagerposition in eine Gebrauchsposition bewegt wird.

12. Medizinische Testvorrichtung nach Anspruch 11, wobei durch ein zweites Niederdrücken des Knopfs die Lanzette gespannt wird.

13. Medizinische Testvorrichtung nach Anspruch 12, wobei durch ein drittes Niederdrücken des Knopfs die Lanzette ausgelöst wird.

14. Medizinische Testvorrichtung nach Anspruch 1, ferner mit einer Kassette, die mindestens zwei Lanzetten enthält, die mindestens teilweise im Gehäuse angeordnet sind, sowie einer Öffnung im Gehäuse, durch die sich die Lanzette erstreckt, wenn sie in der zweiten Position ist, und wobei die Kassette so konfiguriert ist, dass sie sich nach dem Auslösen einer Lanzette so bewegt, dass sie eine nicht benutzte Lanzette auf die Öffnung ausrichtet.

15. Medizinische Testvorrichtung nach Anspruch 14, ferner mit einem Anzeiger, der die Anzahl von nicht benutzten und/oder benutzten Lanzetten in der Kassette anzeigt.

## Revendications

1. Dispositif de test médical, incluant:
un dispositif de test sanguin ayant un logement;
un dispositif de piquage incluant une lancette et une pointe mobile d'une première position située au moins partiellement à l'intérieur du logement jusqu'à une deuxième position située au moins partiellement à l'extérieur du logement ; et
un moyen d'amorçage du dispositif de piquage pour un mouvement de la lancette de la première position jusqu'à la deuxième position.

2. Dispositif de test médical selon la revendication 1, dans lequel le moyen d'amorçage inclut une pointe sur le dispositif de piquage mobile d'une première position jusqu'à une deuxième position.

3. Dispositif de test médical selon la revendication 1, dans lequel le moyen d'amorçage inclut une ouverture dans le logement pour recevoir un élément de test et dans lequel l'insertion d'un élément de test dans l'ouverture amorce la lancette.

4. Dispositif de test médical selon la revendication 1, dans lequel le moyen d'amorçage inclut un couvercle mobile d'une première position sur le logement jusqu'à une deuxième position sur le logement.

5. Dispositif de test médical selon la revendication 1, dans lequel le moyen d'amorçage inclut un levier mobile d'une première position jusqu'à une deuxième position.

6. Dispositif de test médical selon la revendication 1, incluant en outre un indicateur pour indiquer si la lancette est dans l'état amorcé ou non amorcé.

7. Dispositif de test médical selon la revendication 6, incluant en outre un afficheur et dans lequel l'indicateur apparaît sur l'afficheur.

8. Dispositif de test médical selon la revendication 1, dans lequel le moyen d'amorçage inclut un afficheur mobile d'une première position située au moins partiellement à l'intérieur du logement jusqu'à une deuxième position située au moins partiellement à l'extérieur du logement.

9. Dispositif de test médical selon la revendication 1, incluant en outre un afficheur et dans lequel le moyen d'amorçage inclut un couvercle mobile d'une première position sur l'afficheur jusqu'à une deuxième position exposant l'afficheur.

10. Dispositif de test médical selon la revendication 1, dans lequel le moyen d'amorçage inclut un afficheur mobile d'une première position faisant face au logement jusqu'à une deuxième position.

11. Dispositif de test médical selon la revendication 10, dans lequel le moyen d'amorçage inclut un bouton situé sur le logement et l'enfoncement du bouton le déplace d'une position de stockage jusqu'à une position d'utilisation.

12. Dispositif de test médical selon la revendication 11, dans lequel l'enfoncement du bouton une deuxième fois amorce la lancette.

13. Dispositif de test médical selon la revendication 12, dans lequel l'enfoncement du bouton une troisième fois déclenche la lancette.

14. Dispositif de test médical selon la revendication 1, incluant en outre une cartouche contenant au moins deux lancettes situées au moins partiellement à l'intérieur du logement et une ouverture dans le logement à travers laquelle la lancette s'étend lorsqu'elle est dans la deuxième position et dans lequel la cartouche est configurée pour se déplacer après qu'une lancette a été déclenchée afin d'aligner une lancette non utilisée avec l'ouverture.

15. Dispositif de test médical selon la revendication 14, incluant en outre un indicateur affichant le nombre de lancettes non utilisées et/ou utilisées dans la cartouche.
